# EUROPÄISCHE PATENTSCHRIFT

(11) **EP 1 259 120 B1**
(45) Veröffentlichungstag und Bekanntmachung des Hinweises auf die Patenterteilung: **29.08.2007**
(21) Anmeldenummer: 01909798.9
(22) Anmeldetag: 28.02.2001
(51) Int. Cl.: A23J 1/00, C07K 1/14, A61L 27/24

(54) **VERFAHREN ZUR ISOLIERUNG VON KOLLAGEN AUS MARINEN SCHWÄMMEN SOWIE HERSTELLUNG VON NANOPARTIKULÄREM KOLLAGEN UND DESSEN VERWENDUNG**
METHOD FOR ISOLATING COLLAGEN FROM MARINE SPONGE AND PRODUCING NANOPARTICULATE COLLAGEN, AND THE USE THEREOF
PROCEDE D'ISOLATION DE COLLAGENE D'EPONGES MARINES AINSI QUE PRODUCTION DE COLLAGENE NANOPARTICULAIRE ET SON UTILISATION

(30) Priorität: 03.03.2000 DE 10010113
(43) Veröffentlichungstag der Anmeldung: 27.11.2002
(73) Patentinhaber: Schatton, Wolfgang, 65760 Eschborn (DE)
(72) Erfinder: SCHATTON, Wolfgang, 65760 Eschborn (DE); KREUTER, Jörg, 61350 Bad Homburg (DE); MÜLLER, Werner, E., G., 65203 Wiesbaden (DE); SWATSCHEK, Dieter, 89143 Blaubeuren (DE); SCHATTON, Maria, 65760 Eschborn (DE)
(74) Vertreter: Müller, Claudia
(86) Internationale Anmeldenummer: PCT/EP2001/002228
(87) Internationale Veröffentlichungsnummer: WO 2001/064046

(56) Entgegenhaltungen:
- EP-A- 0 218 065
- EP-A- 0 440 198
- EP-A- 0 568 334
- EP-A- 0 901 795
- WO-A-00/69355
- WO-A-92/10287
- US-A- 4 320 201
- DIEHL-SEIFERT B ET AL: "ATTACHMENT OF SPONGE CELLS TO COLLAGEN SUBSTRATA EFFECT OF A COLLAGEN ASSEMBLY FACTOR" JOURNAL OF CELL SCIENCE, Bd. 79, 1985, Seiten 271-286, XP001002686 ISSN: 0021-9533 in der Anmeldung erwähnt
- ROESSLER B ET AL: "COLLAGEN MICROPARTICLES: PREPARATION AND PROPERTIES" JOURNAL OF MICROENCAPSULATION,GB,TAYLOR AND FRANCIS INC. LONDON, Bd. 12, Nr. 1, 1995, Seiten 49-57, XP000486814 ISSN: 0265-2048 in der Anmeldung erwähnt
- CELERIN MARTINA ET AL: "Fungal fimbriae are composed of collagen." EMBO (EUROPEAN MOLECULAR BIOLOGY ORGANIZATION) JOURNAL, Bd. 15, Nr. 17, 1996, Seiten 4445-4453, XP002169423 ISSN: 0261-4189
- SCHENCK S ET AL: "COLLAGENASE PRODUCTION BY NEMATODE TRAPPING FUNGI" APPLIED AND ENVIRONMENTAL MICROBIOLOGY, Bd. 40, Nr. 3, 1980, Seiten 567-570, XP001002731 ISSN: 0099-2240
- VOS P ET AL: "MERKEL CELLS IN-VITRO PRODUCTION OF NERVE GROWTH FACTOR AND SELECTIVE INTERACTIONS WITH SENSORY NEURONS" DEVELOPMENTAL BIOLOGY, Bd. 144, Nr. 2, 1991, Seiten 281-300, XP001024343 ISSN: 0012-1606

## Beschreibung

Die vorliegende Anmeldung betrifft das in den Ansprüchen beschriebene Verfahren zur vereinfachten und damit industriell nutzbaren Isolierung von Schwammkollagen, insbesondere aus marinen Schwämmen, insbesondere aus Schwämmen der Klasse der Chondrosiidae, sowie die Herstellung von Kollagennanopartikeln aus Kollagen.
Die Erfindung betrifft ferner die Verwendung von derartigem Kollagen zur Herstellung eines Mittels zur in-vitro- und in-vivo-Beeinflussung von zellabhängigen Vorgängen.
Insbesondere betrifft die Anmeldung die Verwendung von derart isoliertem Kollagen bzw. von Kollagennano- und -mikropartikeln zur Herstellung von Cremes, Salben, Suspensionen, Tabletten, Kapseln, auch mit verzögerter Freisetzung, Implantate, Pflastern, Schäumen u.a. zur Wundabdeckung, Wirkstoffträger in Parenteralia und Enteralia, Augentropfen, Nanokapseln als Wirkstoffträger und Carrier für Wirkstoffe durch Haut und Schleimhäute sowie Gefäß und Organmembranen.
Das erfindungsgemäß hergestellte Kollagen eignet sich insofern zur Herstellung von Mitteln zur äußeren und inneren vorzugsweise oralen und topischen Anwendung bei der Behandlung entzündlicher, insbesondere auch Cyclooxygenase-abhängiger Erkrankungen in vivo sowie ferner zur Wachstumsförderung von permanenten und neuronalen Zellen in vitro..

Kollagen ist ein bioabbaubares und gut verträgliches Protein und findet als Ausgangsmaterial breite Anwendung in der pharmazeutischen Industrie, Kosmetik und Lebensmittelchemie.

Bislang wurde Kollagen aus Tierhäuten - und Knochen von Schweinen, Kälbern und Rindern gewonnen. Dabei stellte die Möglichkeit, den Anwender durch pathogene Keime, Viren und vor allen Dingen mit BSE (Bovine - spongiforme - Enzephalopathie) zu infizieren, einen entscheidenden Nachteil dar.

Schwämme sind über 600 Millionen Jahre alt und stellen somit die ältesten Vertreter aus der Gruppe der Metazoen dar. Wie alle vielzelligen Tiere besitzen Schwämme das Bindegewebsprotein Kollagen. Schwammkollagen stellt ein wasserunlösliches Protein dar, welches die typische Aminosäurezusammensetzung von bekannten Kollagentypen besitzt. So stellt u.a. Glycin etwa jede dritte Aminosäure dar und die Anteile an Prolin sowie Hydroxyprolin sind hoch..

Schwammkollagen kann nach verschiedenen Methoden isoliert werden. Dabei wurde bislang, wenig praxisnah, Frischmaterial eingesetzt. Das Reinigungsverfahren für derartig isoliertes Schwammkollagen war bislang aus industrieller Sicht meist relativ aufwendig (z.B. Gelfiltration). Auch die Ausbeute war noch nicht zufriedenstellend. So wurden z.B. aus 1000 g Schwamm-Material 21 g Schwammkollagen isoliert.

Dies beschreiben B. Diehl Seufert et al. in J.Cell Sc. 79, 271-85 (1985). Die Isolierung von Kollagen aus dem Schwamm Geodia cydonium und Chondrosia renifornis mit natürlicher Aminosäurezusammensetzung unter Vermeidung von Denaturierungsreagentien erfolgt durch Suspendierung von homogenisiertem frischen Material in Tris-HCl Puffer, pH-Wert Einstellung auf 9, Zentrifugation und Homogenisierung. Die Reinigung erfolgt durch Gelfiltration, wobei wie erwähnt 1,7 bzw. 3,1% Kollagen erhalten werden.

Es besteht daher Bedarf an einem einfachen und wirtschaftlichen Verfahren zur Isolierung von Kollagen aus Schwämmen, wobei die Nachteile für aus tierischem Ausgangsmaterial gewonnenem Kollagen vermieden und gleichzeitig hohe Ausbeuten erzielt werden.

Darüberhinaus ist es von Vorteil, insbesondere kleinstpartikuläres Kollagen herzustellen.

Die Herstellung von Kollagenmikropartikeln wurde bisher für natives Kalbskollagen beschrieben. Es war dabei jedoch nur möglich, Mikropartikel im Grössenbereich von 3 bis 40 µm herzustellen, so wie in B. Rössler, J. Kreuter und D. Scherer Collagen microparticles, preparation and properties, J. Microencapsulation, Vol. 12, No. 1, 49-57 (1995) beschrieben. Dieser Grössenbereich ist für pharmazeutische und kosmetische Anwendungen aber weniger geeignet. Bei einer intravenösen Anwendung besteht die Gefahr einer Embolie, bei der Anwendung auf der Haut oder am Auge wird die Freigabe von daran gebundenen Stoffen verlangsamt, sodass diese nicht innerhalb nützlicher Zeit erfolgt, das heisst, bevor die Zubereitung abgewaschen, bzw. durch Drainage aus dem Auge entfernt wird. Ausserdem kann durch die relativ grosse Teilchengrösse ein unangenehmes kratzendes Gefühl erzeugt werden. Aus diesem Grunde ist es notwendig, den Grössenbereich zu beschränken, das heisst unter 3 µm oder kleiner zu halten.

Aufgabe vorliegender Anmeldung ist es daher, eine produktorientierte Isolierungsmethode für Schwammkollagen zu entwickeln, welche einfach und effektiv in guten Ausbeuten zu einem Produkt mit hohem Kollagenanteil führt, wobei ein minimales toxikologisches Risiko bestehen soll. Gleichzeitig soll das Ausgangsmaterial kostengünstig und praxisnah konserviert sein, so dass dabei auf die Zugabe von tiefgefrorenem Frischmaterial als Ausgangsmaterial verzichtet werden kann (wie es bisher eingesetzt war).

Ferner sollen Kollagenpartikel hergestellt werden, die eine möglichst geringe Grösse, insbesondere im mikro- und nano-Bereich, aufweisen.

Diese Aufgabe wird erfindungsgemäss gelöst, indem man das frische Ausgangsschwammmaterial, welches nicht gefroren sein muss, in Alkohol einlegt, anschliessend mit Wasser wäscht und mit einem Extraktionsmittel, vorzugsweise bei einem pH-Wert von 7-12, insbesondere 8-10, ganz besonders 9-95 bzw. 9,5 versetzt, den resultierenden Kollagenextrakt aufarbeitet. Dies erfolgt vorzugsweise durch Erhöhung des pH-Wertes der Suspension auf pH 8-11, insbesondere 9-10, bevorzugt 9, Rühren, Zentrifugation und anschliessende durch Verringerung des pH-Wertes des Überstands, Zentrifugation und Isolierung des Präzipitats.

Durch diese Vorgehensweise erübrigt sich eine weitere Aufreinigung wie z.B. durch Gelfiltration oder ähnliches. Dabei wird gereinigtes Kollagenprodukt, welches gewünschtenfalls zur Konservierung gefriergetrocknet werden kann, in einer hohen Ausbeute erhalten.

Als Ausgangsmaterial eignen sich die üblichen bekannten, insbesondere marinen Schwämme wie z.B. Demospongiae [Rupert Riedel, Fauna und Flora des Mittelmeeres, Verlag Paul Parey 1983] insbesondere solche mit hohem Kollagenanteil wie z.B. Schwämme der Klasse der Geodiidae, Dysideidae, Spongiidae, Suberitidae, Oscarellidae, Axinellidae und anderen, ganz besonders solches aus der Gruppe der Chondrosiidae, die darüberhinaus ein minimales toxikologisches Risiko aufweisen. Insbesondere bevorzugt sind Chondrosiidae oder auch Axinellidae.

Als Alkohol eignen sich die bekannten geradkettigen oder verzweigtkettigen aliphatischen C₁-C₁₅ Alkohole wie Ethanol, Isopropanol, Butanol, Tert.Butanol, Pentanol, Cyclohexanol, Hexanol etc. Darüberhinaus sind auch Zusätze von aromatischen Alkoholen wie Thymol sowie Benzylalkohol und Kombinationen hiervon, z.B. unter einander oder mit den aliphatischen Alkoholen, insbesondere Ethanol oder Isopropanol, möglich.

Besonders bevorzugt sind C₁-C₄ aliphatische Alkohole, und insbesondere Ethanol. Die Menge an Alkohol richtet sich nach der Menge an Ausgangsmaterial, da dieses in Alkohol eingelegt wird.

Als Extraktionsmittel eignen sich z.B. bekannte Systeme wie z.B. Tris-HCl Puffer (vorzugsweise enthaltend 10 mM EDTA, 8M-urea, 100 mM-2-mercaptoethanol) oder Alkalihaltige Puffersyssteme, z.B. Kaliumkarbonat-haltige, Phosphat-haltige, Stickstoff- bzw. Sulfat-haltige Puffer mit geeignetem pH-Wert. Das Mittel wird dabei im Überschuss bezogen auf das Gewicht des eingesetzten Ausgangsmaterials, insbesondere in der 2-8-fachen Gewichtsmenge, insbesondere der 3-6-fachen und ganz besonders bevorzugt der 5-fachen Menge, eingesetzt.
Solche Puffer sind allgemein bekannt und beispielsweise im Europäischen Arzneibuch beschrieben.

Der pH-Wert kann mit bekannten Mitteln eingestellt bzw. reduziert werden. Hierzu eignen sich z.B. Pufferlösungen wie die oben genannten Stickstoff-, Phosphat, Sulfat-, Kaliumcarbonat-haltigen bzw. organischen Puffer wie z.B. Acetat- oder Citrat-haltige Puffersysteme mit geeignetem pH-Wert.

Das Ausfällen des Kollagens aus dem Überstand nach der Zentrifugation kann durch pH-Wert Reduktion, insbesondere auf Werte zwischen 2 und 6, insbesondere 5-3 und ganz besonders bevorzugt pH 4 durch entsprechende Mittel wie organische Säuren, z.B. Essigsäure (z.B. 100%), Zitronensäure, Ascorbinsäure, Propionsäure, Ameisensäure oder Milchsäure etc. oder durch verdünnte anorganische Säuren, z.B. Salzsäure, Phosphorsäure, Schwefelsäure u.ä. erreicht werden. Die nachfolgende Isolierung des Präzipitats kann durch erneute Zentrifugation erfolgen, wobei vorzugsweise - wie auch bei der anfänglichen Zentrifugation - die Temperatur abgesenkt werden kann, insbesondere auf Werte kleiner 10°C - 1°C, bevorzugt 6°C-1 °C, insbesondere 2°C.

Das erhaltene Produkt (Kollagensediment) ist rein, und kann gewünschtenfalls zur Konservierung gefriergetrocknet werden.

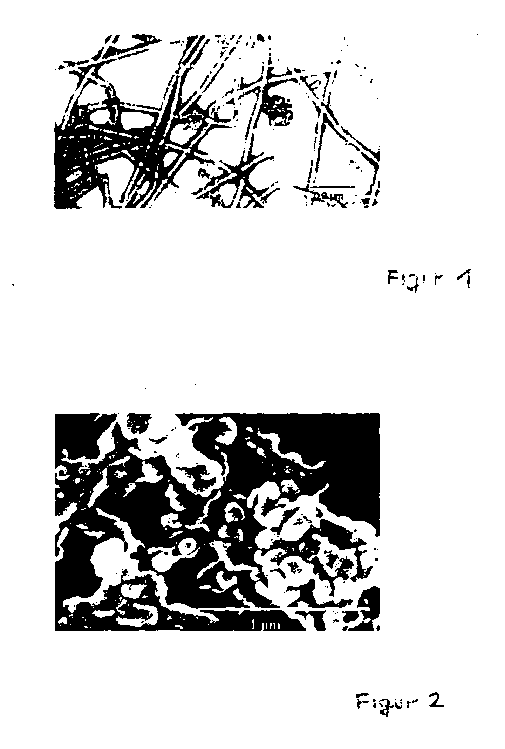

Fig. 1 (vgl. Beispiel 2) zeigt ein solches nach dem erfindungsgemässen Verfahren erhaltenes gefriergetrocknetes Produkt, wobei man die für Kollagen typische periodische Querstreifung der Kollagenfiltration erkennt.

Mit dem erfindungsgemässen Verfahren ist es somit möglich, in hohen Ausbeuten reines Kollagen ohne aufwendige Reinigungsschritte zu erhalten.

Überraschenderweise zeigte sich, dass die für Kollagen typischen Merkmale wie periodische Querstreifung der Fibrillen vorhanden bleibt und damit nativ ist.

Das Verfahren eignet sich insbesondere für ein industrielles 'scaling - up'. Die Reinigung des isolierten Schwammkollagens konnte erfindungsgemäss zugunsten eines praxisorientierten Verfahrens vereinfacht werden. Die Ausbeute an Schwammkollagen liegt mit ca. 10-40 %, durchschnittlich 30% sehr hoch. Da die eingesetzten Schwammspezies aus toxikologischer Sicht nicht bedenklich sind, insbesondere z.B. bei Verwendung essbarer Schwämme aus den Familien der Chondrosiidae oder Axinellidae, kann angenommen werden, dass das so erhaltene Schwammkollagen gut verträglich und aus toxikologischer Sicht für den Menschen weitestgehend unbedenklich ist. Darüber hinaus besitzt das isolierte Schwammkollagen gegenüber Schweine-, Kalbs- und Rinderkollagen vor allem erhebliche Vorteile in Bezug auf die BSE-Problematik.

Wie erwähnt ist es von Vorteil, Kollagenmaterial in besonders kleiner Partikelgrösse herzustellen. Bisher wurde hierzu natives Kalbskollagen emulgiert und mit Glutardialdehydlösung quervernetzt. Die erhaltenen Partikel wiesen eine Grösse von 3-40 µm auf.

Erfindungsgemäss können nun erstmals Partikelgrössen von kleiner 3 µm erzielt werden.

Hierzu wird Kollagen, erhalten nach bisher bekannten Verfahren aus Ausgangsmaterial unterschiedlicher, z.B. tierischer Herkunft wie oben geschildert aus Kalb, Schwein, Rind, oder Schwämmen insbesondere solches erhalten aus Schwämmen nach dem erfindunsgemäßen Verfahren, dispergiert, z.B. in Wasser und zunächst homogenisiert. Der pH-Wert kann vorzugsweise dann oder gegebenenfalls vorzugsweise vor der ersten Homogenisierung auf pH 7-11, insbesondere 8-10, bevorzugt 9-10 insbesondere 9,5 eingestellt und nochmals homogenisiert werden. Anschliessend wird emulgiert, durch Zugabe eines Emulgators und einer Fettphase wie z.B. durch Zugabe von Paraffin und Span ® oder z.B. durch andere W/O-Emulgatoren wie Cetylstearylalkohol, Glycerinmonostearat oder Span ® 85 (Sorbitantrioleat),
oder analoge Produkte wie Arlacel 85, Span 65 (Sorbitantristearat) Arlacel 65, Propylenglykolmonostearat, Sorbitanmonooleat, Span 40, Arlacel 40, Span 20 oder gegebenenfalls O/W-Emulgatoren bekannter Art (Polyethylenglycole mit geeignetem Ethoxylierungsgrad;
wie z.B mit HLB-Wert 8-13, wie z.B. Polyoxyethylenglykol-400--monostearat, -oleylether, -monolaurat,-sorbitanmonolaurat, -sorbitantristearat)
in einer Gewichtsmenge von mehr als der 2 bis 5-fachen Menge der Kollagendispersion. Neben Paraffin eignen sich als Fettphase analoge flüssige Kohlenwasserstoffe wie z.B. Isoparaffin, Dioctylcyclohexan(Cetüol® S), Isohexadecane (Arlamol® HD), Triglyceride wie Miglyol®, Squalan oder Squalen oder Mischungen hiervon.Die Menge an Fettphase ist zusammen mit der Menge an Emulgator angegeben und beträgt wie erwähnt das 2-5-fache der Kollagenmenge.
Anschließend wird das Produkt quervernetzt mit einem Überschuss, nämlich der mehr als doppelten Menge wie bisher, also mindestens der 2 bis 6-fachen Gewichtsmenge, vorzugsweise 3-5-fachen, insbesondere 4-fachen, bezogen auf die eingesetzte Kollagenmenge, an Vernetzungsmittel wie z.B. bifunktionelle Säurechloride, Aldehyde, insbesondere Glutardialdehyd (z.B. eine 25%-ige wässrige Lösung hiervon) oder Maleinsäuredialdehyd oder -dichlorid.

Die Reaktion wird in geeigneter Weise aufgearbeitet, zunächst z.B. durch Zugabe von H₂O₂ abgebrochen. Hierbei wird eine weitaus höhere Gewichtsmenge als bisher eingesetzt, nämlich die 2-6-fache, bevorzugt 3-5-fache, insbesondere 4-fache, bezogen auf die eingesetzte Kollagenmenge. Alternativ können auch andere bekannte geeignete Oxidationsmittel wie z.B. HNO₃ zum Abbruch eingesetzt werden. Die Aufarbeitung erfolgt z.B. durch Zentrifugation, Suspendierung des Sediments in Alkohol, z.B. Isopropanol, erneute Zentrifugation und weitere Durchläufe dieses Zyklus. Im letzten Zyklus erfolgt dann die Suspendierung mit einer schwachen Säure z.B. Ascorbinsäure oder einer anderen organischen Säure, wie z.B. Citronensäure, Milchsäure, Ameisensäure u.ä. Dieses Sediment wird dann erhitzt, z.B. auf Temperaturen > 50°C, insbesondere auf 75°C, weiterhin mehrstündig (z.B. 24) gerührt, zentrifugiert und mit Wasser gewaschen.

Das erhaltene Material kann gefriergetrocknet werden.

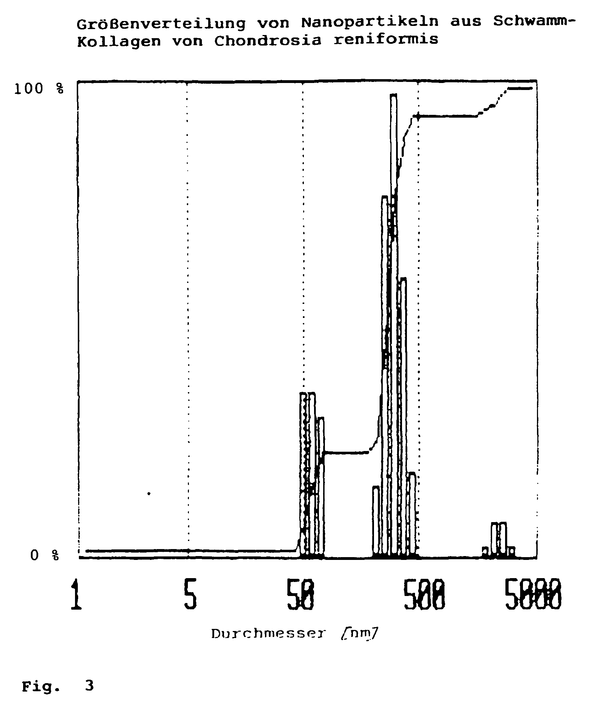

In Fig.3 ist die Größenverteilung von Nanopartikeln, welche wie im nachfolgenden Beispiel 3 beschrieben, hergestellt wurden, dargestellt. Wie hieraus ersichtlich ist, sind mehr als 95% aller Teilchen Nanopartikel.
Überraschenderweise kann auf die oben geschilderte Art kleinstpartikuläres Kollagen hergestellt werden, in dem das Ausgangsmaterial, welches in geeigneter Weise dispergiert wird, z.B. in destilliertem Wasser, zunächst vor der Emulgierung und Vernetzung homogenisiert wird. Die Homogenisierung erfolgt dabei geeigneterweise durch Ultraschallbehandlung, Ultraturrax, oder Hochdruck-Homogenisierer bzw. Micro-Fluidizer. Vorzugsweise kann nach Einstellung des pH-Wertes z.B. mit Kaliumcarbonat von pH 4 auf pH 8 erneut homogenisiert werden. Die Vernetzung erfolgt mit einer höheren als bisher eingesetzten Menge an Vernetzungsmittel.

Besonders bevorzugt ist als Ausgangsmaterial ein Kollagen, erhalten nach dem oben geschilderten erfindungsgemäßen Isolierverfahren aus marinen Schwämmen der Familie der Chondrosiidae.

Dieses neue Verfahren zur Herstellung der Kollagenmikropartikel aus Kollagen, insbesondere aus Schwammkollagen wie z.B. aus marinen Schwämmen der Familie der Chondrosiidae ermöglicht somit die Produktion von Partikeln in einem Grössenbereich von 150 nm bis 3 µm, im Unterschied zu bekannten Verfahren zur Herstellung von Kollagen, das Mikropartikel von nur 3 - 40 µm ermöglicht.

Das auf die erfindungsgemässe Weise erhaltene Kollagen und nanopartikuläre Kollagen findet breite Anwendung in der Pharmazie, Medizin, Kosmetik und Lebensmittelchemie wie z.B. Cremes, Salben, zur Anwendung auf Haut und Schleimhäuten, Suspensionen, Tabletten, Kapseln, auch mit verzögerter Freisetzung, Implantate, Pflastern, Schäumen.

Schwämmen. Vliesen u.a. zur Wundabdeckung, Wirkstoffträger in Parenteralia und Enteralia, Augentropfen, Nanokapseln als Wirkstoffträger und Carrier für Wirkstoffe durch Haut und Schleimhäute sowie Gefäß und Organmembranen. Diese Mittel werden auf bekannte Weise hergestellt. Die Herstellungsverfahren hierfür sind z.B. im DAB beschrieben und bekannt sowohl zur Herstellung von Salben, Cremes als auch von Tabletten etc., wobei in üblichen Mengen gearbeitet wird.
Überraschenderweise zeigte sich, dass das erfindungsgemäß hergestellte Kollagen, insbesondere das nanopartikuläre, eine entzündungshemmende Wirkung insbesondere bei topischer und auch bei oraler neben der intravenösen oder intraperitonealen Anwendung aufweist. Dabei zeigte sich, dass das Mittel Inhibitor von Cyclooxygenase ist sowie eine anti-oxidative Wirkung hat. Besonders überrschend ist dabei die entzündungshemmende Wirkung z.B. bei Gelenkbeschwerden, Arthithis und insbesondere analogen Erkrankungen des Bewegungsapparates, besonders wenn das Mittel oral verabreicht wird. Dazu wird das oben beschriebene insbesondere marine Schwammkollagen als solches oder gefriergetrocknet in einem geeigneten Getränk wie Wasser oder Säften suspendiert und als Getränk verabreicht, z.B. in Mengen von 2-10g, insbesondere 2-6 und bevorzugt 3-5g/Tag. Dabei kann festes also pulverförmiges oder granulietes Kollagen wie unten beschrieben vorzugsweise in einer Menge von 1-10, insbesondere 2-8 und ganz besonders 3-5g/250ml Flüssigkeit, vorzugsweise Wasser vorliegen.
Als topisch verabreichbares Mittel kann sowohl eine Penetrationsverstärkung eines anderen aktiven Mittels bzw. in Kombination hiermit wie z.B. eines Vitamins (Vitamin A,C,E oder deren Mischungen) oder anderen topisch wirksamen Stoffen zur Behandlung von Haut wie Avarol, Avaron oder pflanzliche Extrakte, wie z.B. Extr. Cepae oder Extr. Echinaceae pallidae als auch insbesondere eine Entzündungshemmung festgestellt werden. Insbesondere eignet sich das beschriebene Kollagen zur Behandlung von Hautveränderungen wie z.B. degenerativer Art oder durch äußere oder innere Einflüsse geschädigt, z.B. zur Behandlung von Erythemen nach Sonnenbrand, UV-Bestrahlung oder Verletzungen, oder Psoriasis. Auch für die unten genannten Indikationen dieser Art ist das Mittel geeignet. Das topische Mittel kann in Form von Salben, Cremes, Lotionen bekannter Grundlage oder insbesondere in Form eines Gels wie z. B. eines Hydrogels z.B. auf Basis von Polyacrylat oder eines Oleogels z.B. aus Wasser und Eucerin vorliegen.
Die eingesetzten Oleogele aus einer Wasser- und einer Fettphase basieren insbesondere auf Eucerinum anhydricum, einer Grundlage aus Wollwachsalkoholen und Paraffin, wobei der Anteil an Wasser und der Grundlage variieren kann. Ferner können weitere lipophile Bestandteile zur Beeinflussung der Konsistenz beigefügt sein, wie z.B. Glycerin, Polyethlylenglykole unterschiedlicher Kettenlängen, z.B. PEG400, pflanzliche Öle, wie z.B. Mandelöl, flüssiges Paraffin, Neutralöl u.a..Solche rezepturen sind allgemein bekannt und im DAB10 oder im Europäischen Arzneibuch, aktuelle Ausgabe,z.B.2000 beschrieben. Die Hydrogele können hergestellt werden durch Einsatz von Gelbildner und Wasser, wobei erstere insbesondere ausgewählt werden aus natürlichen Produkten wie CelluloseDerivaten, wie Zellulose-Ester und -Ether, z.B. Hydroxyethyl- Hydroxypropyl-Derivate, z.B. Tylose, oder auch aus sythetischen Produkten wie Polyacrylsäure-Derivate, wie Carbopol oder Carbomer, wie z.B. P934, P940, P941. Sie können nach bekannten Vorschriften, die in den gängigen Arzneibüchern beschrieben sind wie z.B. DA810 oder Europäisches Arzneibuch, aktuelle Ausgabe,z.B.2000, aus alkoholischen Suspensionen durch Basenzusatz für die Gelbildung hergestellt bzw polymerisiert werden.
Die Gele umfassen jeweils pro 100g Gel 0,01-30g, insbersondere 0,01-10g und ganz besonderes 0,01-8g und bevorzugt 0,1-5g Kollagen, also entsprechend 0,01-30;0,01-10; 0, 01-8; 0,1-5%.

Somit kann ein Mittel bereitgestellt werden, welches nicht die Nebenwirkungen der bisher zur Behandlung entzündlicher Erkrankungen eingesetzten Mittel wie z.B. NSDAl's aufweisen und insbesondere oral gut verträglich sind.

Die oben beschriebenen Mittel sind daher geeignet für Zusammensetzungen zur Behandlung von Entzündung in einem Lebewesen und zur Behandlung von anderen entzündungsbedingten Störungen. Beispiele für Entzündungen und entzündungsanaloge Störungen sind Arthritis, einschließlich rheumatoide Arthritis, Wirbelsäulengelenkleiden, Gicht, systemische Lupuserythematose, Osteoarthritis und Jugendarthritis; weiterhin Asthma, Bronchitis, Menstruationskrämpfen, Sehnenentzündung, Bursitis und mit der Haut zusammenhängenden Zuständen wie Psoriasis, Ekzeme, Verbrennungen und Dermatitis. Die beschriebenen Mittel sind auch zur Behandlung von Magen-Darm-Zuständen, wie entzündliche Darmstörung, Crohn's Krankheit, Gastritis, Reizdarmsyndrom und ulcerative Kolitis;zur Behandlung von Entzündung in Erkrankungen wie vasculären Erkrankungen, Periarteritis, Hodgkin'sche Krankheit, Sclerödem, rheumatisches Fieber, Typ I-Diabetis, Myasthenia granvis, Sarcoidose, nephtrotisches Syndrom, Behcet's Syndrom, Polymyositis, Zahnfleischentzündung, Hypersensibilität, Bindehautentzündung, nach Verletzung auftretende Schwellung, Myocardialischämie usw.

Außerdem wird von dieser Erfindung eine Klasse von Zusammensetzungen umfaßt, enthaltend ein wie beschrieben hergestelltes Kollagen, insbesondere marines Schwammkollagen, insbesondere nanopartikuläres, zusammen mit einem oder mehreren nichttoxischen pharmazeutisch verträglichen Trägern und/oder Verdünnungsmitteln und/oder Adjuvantien (zusammenfassend im vorliegenden als "Träger"-materialien bezeichnet) und gegebenenfalls andere Wirkstoffe. Die erfindungsgmäßen Mittel können wie erwähnt verabreicht werden auf jedem geeigneten Weg und in einer Dosis, die für die beabsichtigte Behandlung wirksam ist. Das Mittel kann beispielsweise intravasculär, intraperitoneal, subkutan, intramusculär, insbesondere oral oder topisch verareicht werden.
Zur oralen Verabreichung kann das Mittel in Form von beispielsweise einer Tablette, Kapsel oder Suspension Dabei wird vorzugsweise das-Mittel in Form einer Dosierungseinheit hergestellt.Das Mittel kann außerdem durch Injektion verabreicht werden als eine Zusammensetzung, worin beispielsweise Kochsalzlösung, Dextrose oder Wasser als ein geeigneter Träger verwendet werden können.
Die Menge des verabreichten Mittels und der Dosierungsplan zur Behandlung eines krankhaften Zustandes mit dem beschriebenen Mittel hängen von einer Vielzahl von Faktoren ab, einschließlich dem Alter, Gewicht, Geschlecht und medizinischem Zustand des Patienten, der Schwere der Erkrankung, der Verabreichungsroute und der Häufigkeit der Verabreichung und der jeweiligen verwendeten Verbindung, und kann deshalb.weitestgehend schwanken. Die erfindungsgemäßen Mittel können gewöhnlich kombiniert mit einem oder mehreren Adjuvantien, die sich für die angegebene Verabreichungsroute eignen. Wenn per os verabreicht wird, kann das beschriebene Kollagenprodukt auch vermischt werden mit Lactose, Saccharose, Stärkepulver, Celluloseester oder Alkansäuren, Cellulosealkylester, Talk, Stearinsäure, Magnesiumstearat, Magnesiumoxid, Natrium- und Calciumsalzen von Phosphor- und Schwefelsäuren, Gelatine, Akaziengummi, Natriumalginat, Glykole Polyvinylpyrrolidon und/oder Polyvinylalkohol und dann tablettiert oder verkapselt werden zur einfachen Verabreichung. Solche Kapseln oder Tabletten können eine kontrollierte Freigabeformulierung enthalten, wie sie gegeben sein kann in einer Dispersion des Mittels in Hydroxypropylmethylcellulose. Formulierungen für die parenterale Verabreichung können in Form von wäßrigen oder nicht-wäßrigen isotonischen sterilen Injektionslösungen oder Suspensionen vorliegen. Diese Lösungen und Suspensionen können hergestellt werden aus sterilen Pulvern oder Granulaten mit einem oder mehreren der Träger oder Verdünnungsmittel, wie sie genannt wurden für die Verwendung in den Formulierungen für orale Verabreichung. Die Mittel können gelöst sein in Wasser, Polyethylenglykol, Propylenglykol, Ethanol, Maisöl, Baumwollsamenöl, Erdnußöl, Sesamöl, Benzylalkohol, Natriumchlorid und/oder verschiedenen Puffern. Andere Adjuvantien und Arten der Verabreichung sind bekannt.
Als Mittel zur Beeinflüssung von zellabhängigen Vorgängen in vitro wird das oben beschriebene Kollagen in der Zellsuspension in einer Menge von 0,01-150µg/ml, insbesondere 0,1-100µg/ml und ganz besonders 1-60µg/ml Zellsuspension eingesetzt. Als Zellen zur Wachstumsförderung eignen sich besonders permanente Zellen wie CEM-Zellen oder neuronale Zellen wie kortikale Zellen. Als Kollagen eignet sich entweder das direkt isolierte wie oben beschrieben oder das erfindungsgemäß nanopartikuläre gemäß vorliegender Anmeldung.Das Kollagen wird dabei entweder in fester Form der Zellprobe zugefügt und liegt als Suspension vor oder es wird in alkalischen wäßrigem Milieu (pH-Wert größer 7, insbesondere größer 8) gelöst, in das Probengefäß gegeben, sodann angesäuert, bis das Kollagen ausfällt und dann die Zellprobe hinzugesetzt.

Die Erfindung wird anhand der nachfolgenden Beispiele näher erläutert.

### Beispiel 1

### Verfahren zur Isolierung von Schwammkollagen aus dem marinen Schwamm der Familie Chondrosiidae

Das Schwamm-Material wird gesammelt, in grobe Stücke zerkleinert und sofort zur Konservierung in Ethanol eingelegt. Die Schwammstücke werden dreimal mit Aqua dest. gewaschen und mit einem Mixer homogenisiert. Das zerkleinerte Material wird mit der fünffachen Gewichtsmenge an Extraktionsmittel (pH 9:5; 10 mM-EDTA, 8. M-urea, 100 mM-2-mercaptoethanol) versetzt. Der pH-Wert der resultierenden dunkelbraunen Suspension wird mit Tris von pH 7 auf pH 9 eingestellt. Diese Suspension wird 24 h bei Raumtemperatur gerührt und anschliessend zentrifugiert (5000***g***, 6 min, 2°C). Das Pellet wird verworfen. Der pH-Wert des Überstandes wird mit Essigsäure 100% auf pH 4 eingestellt. Es bildete sich ein Präzipitat, das gesammelt wird (20000 ***g***, 20 min, 2°C). Das Pellet wird mit Wasser versetzt und nochmals zentrifugiert (20000 ***g***, 10 min, 2°C). Man erhält einen klaren, leicht gefärbten Überstand sowie das gereinigte Kollagensediment, welches zur Konservierung gefriergetrocknet werden kann.

Die Ausbeute an Kollagen liegt bei etwa 30 % (gefriergetrocknetes Kollagensediment im Verhältnis zu gefriergetrocknetem Schwamm-Material).

### Beispiel 2

### Elektronenmikroskopische Charakterisierung des isolierten Schwammkollagens (TEM / Transmissionselektronenmikroskopie)

Die gegfriergetrocknete Kollagenprobe wird zur Untersuchung mit einer 2% igen Phosphorwolframsäure nach der sogenannten single droplet Methode negativ kontrastiert (vgl. J.R. Harris, Negative staining and cryoelectron microscopy. The thin film techniques. Royal Microscopical Society Microscopy Handbook No. 35. BIOS Scientific Publishers Ltd., Oxford, U.K.)

In der Vergrösserung erkennt man die für Kollagen typische, periodische Querstreifung der Kollagenfibrillen in der TEM-Aufnahme gemäss Fig. 1

### Beispiel 3

### Herstellung von Kollagenmikropartikeln aus Schwammkollagen der Familie Chondrosiidae

Zunächst wird eine 0,75 % ige Schwammkollagen-Dispersion hergestellt, indem das gefriergetrocknete Material mit destilliertem Wasser versetzt und mit einem Ultra-Turrax homogenisiert wird. Der pH-Wert der Dispersion wird mit Kaliumcarbonat auf pH 9,5 eingestellt. Anschliessend wird die Dispersion ein zweites Mal mit dem Ultraturrax homogenisiert.

Man stellt eine Mischung aus 10 g Span^{®} und 250 g flüssigem Paraffin her und fügt 85 ml der 0,75 % igen Kollagendispersion hinzu. Diese Mischung wird 10 Minuten bei etwa 6000 rpm mit einem Ultraturrax (Ika-Werk, Staufen, Germany) emulgiert. Anschliessend wird die Emulsion ununterbrochen mit einem Flügelrührer gerührt. Zur Quervernetzung der Kollagenketten gibt man 12 g einer 25 % igen wässrigen Glutardialdehyd-Lösung hinzu. Man beendet diese Reaktion nach 12 Minuten durch Zugabe von 16 g einer 30 % igen wässrigen Wasserstoffperoxid-Lösung. Danach lässt man den Ansatz weitere 15 Minuten rühren. Die Emulsion wird mit 100 ml 2-propanol verdünnt.

Zur Reinigung der CMPs wird die Emulsion zentrifugiert (30 Minuten / 10444 g), der ölige Überstand verworfen und das Sediment in 50 % 2-propanol suspendiert. Anschliessend wird die Suspension wieder zentrifugiert. Dieser Reinigungsschritt wird wiederholt. Das erhaltene CMP-Sediment wird in einer 4 % igen wässrigen Ascorbinsäure-Lösung suspendiert. Man erhitzt diese Suspension (75° C, 30 Minuten), um Restmengen an oxidierenden Substanzen zu zerstören. Der Ansatz wird für 24 h auf einem Magnetrührer bei Raumtemperatur gerührt. Anschliessend werden die CMP durch Zentrifugation (30 Minuten, 10444 g) gereinigt und anschliessend zweimal mit Wasser gewaschen.

Das Material wird gefriergetrocknet. Die Ausbeute an CMP liegt bei etwa 10 %.

### Beispiel 4

### Rasterelektronenmikroskopische Untersuchung der Kollagenmikropartikel gemäss Beispiel 3

Die Kollagenmikropartikel wurden mit einem etwa 4 nm dicken Platinfilm beschichtet und mit dem Rasterelektronenmikroskop SE 4500 Hitachi S untersucht. Die Grösse der sphärischen Kollagenpartikel liegt bei 120 bis 300 nm (Fig. 2)

### Beispiel 5

### Partikelgrössenbestimmung / Photonenkorrelationsspektroskopie (PCS) von Kollagenpartikeln gemäß Beispiel 3

Die Partikelgrössenbestimmung wurde mit Hilfe der Photonenkorrelationsspektroskopie (PCS) durchgeführt. Das gefriergetrocknete Schwammkollagen wurde in gefiltertem Aqua bidest. (pH 9.8, eingestellt mit K₂CO₃) vor der Messung resuspendiert. Es ergab sich eine Partikelgrössenverteilung von 150 nm bis 3 µm. Während sich die untere Grenze recht gut mit den Ergebnissen der Rasterelektronenmikroskopie deckt, liegt der obere Bereich mit 3 µm deutlich über dem Wert von 300 nm. Dies könnte an Agglomeraten liegen, die sich beim redispergieren in Wasser bilden und sich auch im Ultraschallbad nicht auflösen lassen.

In Fig.3 ist die Größenverteilung der gemäß beispiel 3 hergestellten und in Fig.2 gezeigten Nanopartikel dargestellt. Wie hieraus ersichtlich ist, sind mehr als 95% aller Teilchen Nanopartikel, 25% aller Teilchen weisen einen mittleren Durchmesser von 50nm auf und mehr als 70% liegen im Bereich von 300nm (Abzisse=Durchmesser der Teilchen [nm], Ordinate=%Anteile).

### Beispiel 6:

### Thio-Barbitursäure (TBA) - Antioxydanstest mit Kollagen aus Chondrosia reniformis

Der Test beruht auf der Bildung eines bei 532 nm meßbaren Reaktionsproduktes von Thio-Barbitursäure (TBA) mit Malondialdehyd (MDA), das u.a. beim oxidativen Abbau von mehrfach ungesättigten Fettsäuren, wie sie im Trilinolenin (Sigma) enthalten sind, entsteht.

Trilinolenin und die Versuchssubstanzen, Schwammkollagen aus Chondrisa reniformis hergestellt wie in Beispiel 1 beschrieben und Butylhydroxytoluol [BHT] werden in DMSO gelöst.
400 µl Trililonenin werden in 2 ml HAM F10 - Medium 4 Stunden lang bei 37° C in Gegenwart von DMSO als Kontrolle oder DMSO - haltigen Substanzlösungen inkubiert.

Zur Bestimmung des gesamten TBA-reaktiven Materials werden 1,0 ml des Inkubationsansatzes entnommen und mit 1,5 ml TBA- Lösung (0,67% in 0,05 M NaOH) sowie 1,5 ml Tri-chloressigsäure (20%) gemischt.
Nach 60 minütiger Inkubation in einem kochenden Wasserbad werden die Proben abgekühlt und 10 Minuten bei 2000 U/Min zentrifugiert. Die optische Dichte wird bei 532 nm bestimmt. Frisches Tetramethoxipropan, aus dem unter den Versuchsbedingungen MDA entsteht dient als Eichstandard. Die Ergebnisse werden als µmol MDA - Equivalente berechnet. Es werden Dreifachbestimmungen durchgeführt.

**Tabelle1:** Wirkung von Schwammkollagen im Thio-Barbitursäure (TBA) - Antioxydanstest (% Hemmung (± SD) der Bildung von TBA reaktivem Material im Vergleich mit BHT als Standardsubstanz)

**Tabelle 1**

| Substanz | | Konzentration [µM] | | |
|---|---|---|---|---|
| | 5 | 10 | 20 | 50 |
| | | | | |
| BHT | 71 (±1,5) | 73 (±2,1) | 79 (±0,3) | 85 (±0,5) |
| Kollagen* | 18 (±1,3) | 37 (±2,3) | 41 (±3,9) | 47 (±1,8) |

| | | | | |
|---|---|---|---|---|
| *Kollagen = Kollagen aus Chondrosia reniformis | | | | |

Überraschenderweise hemmt Kollagen aus Chondrosia reniformis die Bildung von TBAreaktivem Material. Dies war bisher nicht bekannt. Die Wirkungsstärke beträgt etwa 50% der Standardsubstanz BHT. Kollagen aus Chondrosia reniformis zeigt im geprüften Konzentrationsbereich mittelstarke Wirkungen mit flachverlaufender Dosiswirkungsbeziehung.

### Beispiel 7:

### In vitro-Hemmung der Cyclooxygenase.

Die Cyclooxygenaseaktivität wurde in Monozyten in Gegenwart oder Abwesenheit des Agonisten Lipopolysaccharid [LPS] (L-4130; Sigma, St.Louis) bestimmt. Hierzu wurden menschliche Monozyten in einer Dichte von 106-Zellen pro ml in RPMI-1640-Medium für 24 h gehalten. Anschließend wurden 30 µM Arachidonsäure zu den Kulturen gegeben und die Enzymaktivität 30 Minuten später anhand der Produktionsrate von Prostaglandin E2 gemessen.

### Tabelle2:

Effekt von Schwammkollagen, herstellt wie in Beispiel 1 beschrieben, auf die Cyclooxygenaseaktivität in menschlichen Monozyten.
Die Werte stellen Mittelwerte (± S.D.) von fünf unabhängigen Ergebnissen dar.

**Tabelle 2**

| Zusatz | Kollagen | Cyclooxygenase-Aktivität |
|---|---|---|
| von LPS | (µg/ml) | (pg PGE2/106 Zellen) |
| kein | 0 | 88 ± 10 |
| | 1 | 71 ± 9 |
| | 5 | 52 ± 6 |
| LPS | 0 | 243 ± 22 |
| | 0.5 | 187 ± 19 |
| | 1.0 | 79 ± 7 |
| | 5.0 | 61 ± 7 |

### Ergebnis:

Nach Zusatz von LPS kommt es zu einer Steigerung der Cyclooxygenaseaktivität von 88 auf 243 pg PGE 2 pro 106 Zellen innerhalb von 30 Minuten. Steigende Konzentrationen von Schwammkollagen aus Chondrosia reniformis heben den stimulierenden Effekt von LPS auf die Cyclooxygenase vermehrt auf. Bei einer Konzentration von 3 µg/ml wird die Induktion der Cyclooxygenase auf 61% herabgesetzt.

### Beispiel 8:

### Schwammkollagen bei chronisch-entzündlichen und degenerativen Bewegungsstörungen

Der Schwamm Chondrosia reniformis wird noch in heutiger Zeit gegessen. 10 Patienten mit chronischer Arthritis stimmten daher der oralen Behandlung zu, 5g Kollagen aus Chondrosia reniformis, hergestellt wie in Beispiel 1 beschrieben, über einen Zeitraum von 4 Wochen dreimal täglich suspendiert in einem Getränk einzunehmen. Alle Patienten berichteten übereinstimmend nach kurzer Zeit von einem Rückgang der Schmerzen in den Gelenken und von einer verbesserten Beweglichkeit.

### Beispiel 9:

### Penetration der Haut nackter Mäuse in-vitro

Aus dem Kollagen von Chondrosia reniformis gewonnene Nanopartikel, hergestellt wie in Biespiel 3 beschrieben, wurden mit 14C-markierten Retinol beladen [Beladungsrate 15%] und in ein Oleogel aus Wasser/Eucerin wie oben beschrieben (Kollagen:1 %, als 10%ige Suspension zugesetzt) eingearbeitet.

Diffusionszellen nach Franz [n=6] wurden mit physiologischer Salzlösung gefüllt und die Akzeptorzellen mit einem frisch präparierten Stück Haut von Nacktmäusen abgedeckt. 100mg des mit 14C-markierten Retinol beladenen Gels wurden auf die Hautstücke aufgebracht. Als Kontrollen [n=6] dienten entsprechende Gele mit 14C-markiertem gelösten Retinol.
Nach 2, 4, 8, 16, 24 und 36 Stunden wurde die 14C-Aktivität in der Akzeptorzelle bestimmt.

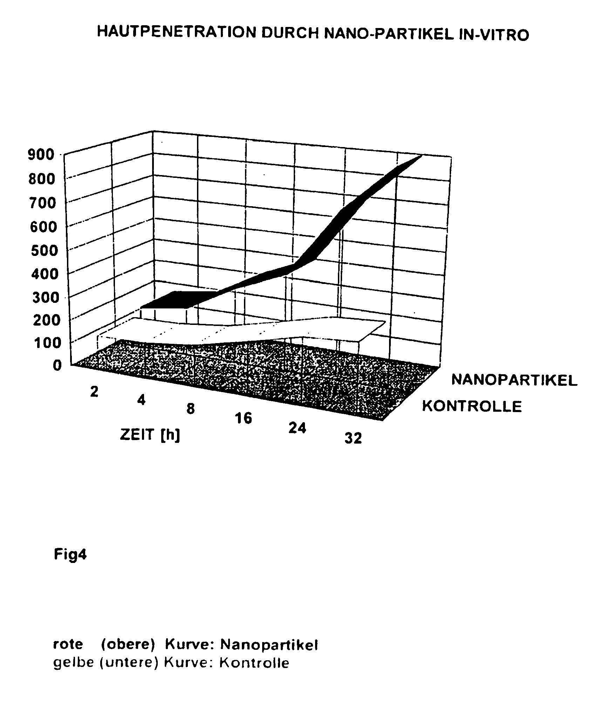

Die Ergebnisse sind in Figur 4 dargestellt und belegen deutlich die Überlegenheit von Schwammkollagen-Nanopartikeln in der Penetration von Mäusehaut im Vergleich mit einem Hydrogel, das die markierte Substanz nur in gelöster Form enthält.

### Beispiel 10:

### Untersuchungen am UV- oder Hitze-induzierten Erythem der menschlichen Haut

Die Untersuchungen wurden an freiwilligen Probanden durchgeführt, die akut unter Sonnenbrand [n=7] oder Verbrennungen [n=3] litten. Schwammkollagen-Nanopartikel [1 %] gemäß Beispiel 3 in einem Polyacrylatgel, hergestellt wie oben beschrieben (1%Kollagen, als 10%ige Suspension zugesetzt) wurden topisch verabfolgt.
Die klinische Wirkung des applizierten Gels auf die UV- oder Verbrennungs-bedingte Veränderung der Haut wurde 1 Stunde nach der großflächigen Applikation des Gels, sowie 2, 4, 6 und 24 Stunden später makroskopisch beurteilt und die Befindlichkeit der Patienten abgefragt.

### Ergebnis:

Schwammkollagen verminderte das Erythem rasch während den ersten Stunden nach dem Erythemauslösenden Ereignis. Die einmalige Applikation besaß eine langanhaltende Hemmwirkung bis zu 5 Stunden. Diese konnte dadurch verstärkt werden, daß bekannte topisch Entzündungshemmende Stoffe in die Nanopartikel eingearbeitet wurden, wie z.B. Vitamin E, Avarol, Avaron oder pflanzliche Extrakte, wie z.B. Extr. Cepae oder Extr. Echinaceae pallidae.

### Beispiel 11

### Toxizitätsuntersuchungen am erfindungsgemäßen Kollagen

Zur Bestimmung der Toxizität wurden die Einflüsse von Schwammkollagen auf das zelluläre Wachstum in Kultur wurde an einer permanenten Zellinie und an frisch präparierten neuronalen Zellen untersucht.

### 1. CEM-Zellen (permanente menschliche Leukämie-Zellen)

CEM-Zellen [Sechoy et al., Exp. Cell Res. 185: 122, 1989 und Avramis et al., AIDS 3: 417, 1989] wurden entweder nicht mit Schwammkollagen behandelt (Kontrollprobe) oder mit unterschiedlichen Konzentrationen hiervon inkubiert.
Untersuchungsparameter war das Zellwachstum. CEM-Zellen wurden in einer Konzentration von 0,2 x 10⁸ Zellen/ml Kultur-Medium zum Beimpfen der Kultur verwendet. Nach 4-tägiger Inkubation betrug die Dichte der CEM-Zellen 1,9 x 10⁸ Zellen/ml. Dieser Wert bildete den Kontrollwert. Nach weiteren 4 Tagen wurde die Zelldichte erneut bestimmt.

### 2. Neuronale Zellen

Kortikale Zellen (Neurone) wurden aus Gehirnen von neugeborenen Wistar-Ratten präpariert und unter Zellkulturbedingungen gehalten. Als Kulturmedium wurde MEM-Medium (mit 10% Pferdeserum) verwendet und bei einer 90%igen Luft- und 10%igen CO2-Atmosphäre inkubiert. Nach gewöhnlich 48 Stunden Inkubation in Kultur wurden die Zellen für die Experimente eingesetzt. Die Kulturen enthalten in überwiegender Konzentration (über 70%) Neurone und etwa 20% GFAP-positive Astrozyten (Müller et al.: Europ. J. Pharmacol. - Molec. Pharmacol. Sect. 226: 209-214, 1992). Nach weiteren 4 Tagen wurde sodann die Zelldichte im Versuch gemessen.
Die Ergebnisse sind in Tabelle 3 dargestellt:

**Tabelle 3:**

| | | Konzentration des Schwammkollagens (µg/ml) | Zellkonzentration x 1.000.000/ml |
|---|---|---|---|
| Kontrolle | | 0 | 1,9 ± 0,24 |

| 1. | CEM-Zellen | | |
|---|---|---|---|
| | | 0,1 | 2,0 ± 0,2 |
| | | 1,0 | 2,5 ± 0,3 |
| | | 1 0,0 | 2,9 ± 0,3 |
| | | 100,0 | 2,7 ± 0,2 |
| | | | |

| 2. | Neuronale Zellen | | |
|---|---|---|---|
| | | 0,1 | 2,3 ± 0,2 |
| | | 1,0 | 2,7 ± 0,3 |
| | | 10,0 | 3,4 ± 0,2 |
| | | 100,0 | 2,5 ± 0,2 |

Die Ergebnisse der Tabelle stellen Mittelwerte von 6 parallelen Experimenten mit den Standardabweichungen dar. Die Signifikanz wurde durch dem Student's t-Test ermittelt (Sachs, L.: Angewandte Statistik. Berlin: Springer-Verlag; pp. 209-216; 1984).

Es ist ersichtlich, daß Schwammkollagen keine negativen Auswirkungen auf die Wachstumsraten von permanenten Zellen und neuronalen Zellen in primärer Zellkultur ausübt. Andererseits steigern CEM-Zellen und Neuronale Zellen in Gegenwart von Schwammkollagen ihre Wachstumsraten über Kontrollwerte significant (p < 0.001). Erst bei Konzentrationen um 100 µg/ml kommt es zu keiner weiteren Wachstumssteigerung.

Hieraus ist ersichtlich, dass das erfindungsgemäße Kollagen einerseits auf z.B.Monocytenabhängige Zellen und hiervon ableitbare Enzyme wie Cycloxygenase inhibierend wirkt, ohne toxisch zu sein und andererseits auf permanente Zellen und neuronale Zellen wachstumsfördernd wirkt. Somit ist erfindungsgemäß hergestelltes Kollagen nicht nur zur in-vivo-Behandlung besonders Cyclooxygenase-abhängiger Erkrankungen verwendbar, sondern insbesondere auch zur vorzugsweise in vitro-Beeinflussung permanenter oder neuronaler Zellen. Es kann hierbei eingesetzt werden in den oben angegebenen Mengen, insbesondere zwischen 0,01, bevorzugt 0,1 und 100µg/ml Zellsuspension.

## Patentansprüche

1. Verfahren zur Isolierung von Kollagen aus marinen Schwämmen, **dadurch gekennzeichnet, dass** man das Ausgangsmaterial in Alkohol einlegt, anschließend wäscht, mit einem Extraktionsmittel behandelt und den so erhaltenen Kollagenextrakt aufarbeitet.

2. Verfahren nach Anspruch 1, **dadurch gekennzeichnet, dass** man Schwammkollagen aus Demospongiae, bevorzugt Chondrosiidae, isoliert.

3. Verfahren nach einem der Anspruch 1 oder 2, **dadurch gekennzeichnet, dass** man als Alkohol Ethanol verwendet

4. Verfahren nach einem der Ansprüche 1 bis 3, **dadurch gekennzeichnet, dass** man als Extraktionsmittel basische Puffersysteme, bevorzugt Tris- Puffer, verwendet.

5. Verfahren nach einem der Ansprüche 1 bis 4, **dadurch gekennzeichnet, dass** man das Extraktionsmittel in einem Überschuss, bezogen auf die Gewichtsmenge an Schwamm- Ausgangmaterial einsetzt.

6. Verfahren nach einem der Ansprüche 1 bis 5, **dadurch gekennzeichnet, dass** man zur Aufarbeitung den pH- Wert des Kollagenextraktes erhöht, die Suspension rührt, zentrifugiert, den Überstand ansäuert und das Präzipitat isoliert.

7. Verfahren nach einem der Ansprüche 1 bis 6, **dadurch gekennzeichnet, dass** man das isolierte Kollagenprodukt gefriergetrocknet.

8. Verfahren gemäß einem der Ansprüche 1 bis 7, **dadurch gekennzeichnet, dass** man das Ausgangsschwammmaterial in Alkohol einlegt, anschließend mit Wasser wäscht, und mit einem Extraktionsmittel bei einem pH- Wert von 7-12 versetzt und den resultierenden Kollagenextrakt aufarbeitet.

9. Natives, bei pH 8-11 im Überstand einer Zentrifugation vorliegendes Kollagen aus Schwämmen, erhältlich nach dem Verfahren gemäß Anspruch 8.

10. Kollagen gemäß Anspruch 9 zur Anwendung in der Pharmazie, Medizin, Kosmetik, Lebensmittelchemie.

11. Verwendung eines Kollagenproduktes, hergestellt nach dem Verfahren gemäß einem der Ansprüche 1 bis 8 zur Herstellung von Cremes, Salben, Suspensionen, Tabletten, Kapseln, Implantaten, Pflastern, Schäumen, Wirkstoffträgern in Parenteralia und Enteralia, Augentropfen, Nanokapseln für Wirkstoffträger, Carrier für Wirkstoffe durch Haut, Schleimhäute, Gefäß und Organmembranen.

12. Verfahren zur Herstellung von nanopartikulärern Kollagen aus marinen Schwämmen, **dadurch gekennzeichnet, dass** man als Ausgangskollagenmaterial ein Kollagenprodukt, hergestellt nach dem Verfahren gemäß einem der Ansprüche 1 bis 8, dispergiert und homogenisiert, anschließend emulgiert und mit einem Überschuss an Vernetzungsmittel vernetzt und anschließend aufarbeitet.

13. Verfahren nach Anspruch 12, **dadurch gekennzeichnet, dass** man Kollagen, erhalten aus den Schwämmen der Klasse Chondrosiidae einsetzt.

14. Verfahren nach einem der Ansprüche 12 bis 13, **dadurch gekennzeichnet, dass** man als Vernetzungsmittel Glutardialdehyd einsetzt.

15. Nanopartikuläres Kollagen aus Schwämmen, erhältlich nach dem Verfahren gemäß einem der Ansprüche 12 bis 14.

16. Kollagen gemäß Anspruch 15 zur Anwendung in der Pharmazie, Medizin, Kosmetik, Lebensmittelchemie.

17. Verwendung eines Kollagenproduktes gemäß Anspruch 15 oder hergestellt gemäß Anspruch 12-14 zur Herstellung von Cremes, Salben, zur Anwendung auf die Haut und Schleimhaut, Suspensionen, Tabletten, Kapseln, Implantate, Pflastern, Schäumen, Schwämmen, Vliesen, zur Wundabdeckung, Nanokapseln als Wirkstoffträger, Wirkstoffträger in Parenteralia und Enteralia, Augentropfen, Carrier für Wirkstoffe durch Haut, Schleimhäute, Gefäß und Organmembranen.

18. Verwendung gemäß Anspruch 17 zur Herstellung eines oral verabreichbaren Mittels zur Behandlung von Erkrankungen des Bewegungsapparates.

19. Verwendung gemäß Anspruch 17 zur Herstellung eines topisch verabreichbaren Mittels in Form eines Oleo- oder Hydrogels.

20. Verwendung eines Kollagenproduktes hergestellt gemäß Anspruch 1-8 oder 12-14 oder des Kollagenprodukts gemäß Anspruch 9 oder 15 als biochemisches Mittel zur in vitro- wachstumsfördemden Anwendung bei permanenten und neuronalen Zellen.

21. Gel in Form eines Oleogels oder Hydrogels, enthaltend Wasser, Gelgrundlage sowie natives Kollagen aus Schwämmen gemäß Anspruch 9 oder hergestellt nach dem Verfahren gemäß einem der Anspruche 1 bis 8 oder daraus erhaltenes Nanokollagen gemäß Anspruch 15 oder hergestellt gemäß den Ansprüchen 12 bis 14.

22. Kollagensuspension, enthaltend natives Kollagen gemäß Anspruch 9 oder hergestellt nach dem Verfahren gemäß einem der Ansprüche 1 bis 8, oder daraus erhaltenes Nanokollagen gemäß Anspruch 15 oder hergestellt gemäß Anspruch 12 bis 14, in Wasser in einer Menge von 1-10 g Kollagen /250 ml.

## Claims

1. A method for isolating collagen from marine sponges, **characterised in that** the starting material is placed in alcohol, subsequently washed, treated with an extractant, and the resultant collagen extract is then further processed.

2. A method according to claim 1, **characterised in that** sponge collagen from Demospongiae, preferably Chondrosiidae is isolated.

3. A method according to either of claim 1 or claim 2, **characterised in that** ethanol is used as the alcohol.

4. A method according to any one of claims 1 to 3, **characterised in that** basic buffer systems, preferably tris buffers, are used as the extractant.

5. A method according to any one of claims 1 to 4, **characterised in that** the extractant is used in excess relative to the weight of sponge starting material.

6. A method according to any one of claims 1 to 5, **characterised in that**, for the purpose of further processing, the pH value of the collagen extract is increased, the suspension is stirred, centrifuged, the residue is acidified and the precipitate is isolated.

7. A method according to any one of claims 1 to 6, **characterised in that** the isolated collagen product is freeze-dried.

8. A method according to any one of claims 1 to 7, **characterised in that** the sponge starting material is placed in alcohol, subsequently washed with water and combined with an extractant at a pH value of 7-12 and the resultant collagen extract is further processed.

9. Native collagen from sponges, present at pH 8-11 in a centrifugation supernatant, obtainable by the method according to claim 8.

10. Collagen according to claim 9 for use in the pharmaceutical, medical, cosmetics and food chemistry sectors.

11. Use of a collagen product, produced by the method according to any one of claims 1 to 8, for producing creams, ointments, suspensions, tablets, capsules, implants, dressings, foams, active ingredient carriers in parenteral and enteral preparations, eye drops, nanocapsules for active ingredient carriers, carriers for active ingredients through the skin, mucous membranes, vessels and organ membranes.

12. A method for producing nanoparticulate collagen from marine sponges, **characterised in that** a collagen product, produced by the method according to any one of claims 1 to 8, as the starting collagen material is dispersed and homogenised, subsequently emulsified and crosslinked with an excess of crosslinking agent, and subsequently further processed.

13. A method according to claim 12, **characterised in that** collagen obtained from sponges of the class Chondrosiidae is used.

14. A method according to either one of claims 12 to 13, **characterised in that** glutardialdehyde is used as the crosslinking agent.

15. Nanoparticulate collagen from sponges obtainable by the method according to any one of claims 12 to 14.

16. Collagen according to claim 15 for use in the pharmaceutical, medical, cosmetics and food chemistry sectors.

17. Use of a collagen product according to claim 15 or produced according to claims 12-14 for producing creams, ointments for use on the skin and mucous membranes, suspensions, tablets, capsules, implants, dressings, foams, sponges, nonwovens, for covering wounds, nanocapsules as active ingredient carriers, active ingredient carriers in parenteral and enteral preparations, eye drops, carriers for active ingredients through the skin, mucous membranes, vessels and organ membranes.

18. Use according to claim 17 for producing an orally administrable agent for treating diseases of the locomotor system.

19. Use according to claim 17 for producing a topically administrable agent in the form of an oleogel or hydrogel.

20. Use of a collagen product produced according to claims 1-8 or 12-14 or the collagen product according to claim 9 or claim 15 as a biochemical agent for *in vitro* growth-enhancing use in permanent and neuronal cells.

21. A gel in the form of oleogel or a hydrogel containing water, a gel base together with native collagen from sponges according to claim 9 or produced by the method according to any one of claims 1-8 or nanocollagen obtained therefrom according to claim 15 or produced according to claims 12 to 14.

22. A collagen suspension containing native collagen according to claim 9 or produced by the method according to any one of claims 1-8 or nanocollagen obtained therefrom according to claim 15 or produced according to claims 12 to 14 in water in a quantity of 1-10 g of collagen/250 ml.

## Revendications

1. Procédé pour isoler du collagène à partir d'éponges marines, **caractérisé en ce que** l'on dépose la matière de départ dans de l'alcool, on la lave ensuite, on la traite avec un agent d'extraction et on traite l'extrait de collagène ainsi obtenu.

2. Procédé selon la revendication 1, **caractérisé en ce que** l'on isole du collagène d'éponge de Demospongiae, de préférence de Chondrosiidae.

3. Procédé selon l'une quelconque des revendications 1 ou 2, **caractérisé en ce que** l'on utilise comme alcool de l'éthanol.

4. Procédé selon l'une quelconque des revendications 1 à 3, **caractérisé en ce que** l'on utilise comme agent d'extraction des systèmes tampons basiques, de préférence tris-tampon.

5. Procédé selon l'une quelconque des revendications 1 à 4, **caractérisé en ce que** l'on utilise l'agent d'extraction dans un excès rapporté à la quantité de masse de la matière de départ d'éponge.

6. Procédé selon l'une quelconque des revendications 1 à 5, **caractérisé en ce que** l'on élève la valeur du pH de l'extrait de collagène pour le traitement, on agite la suspension, on centrifuge, on acidifie le résidu et on isole le précipité.

7. Procédé selon l'une quelconque des revendications 1 à 6, **caractérisé en ce que** l'on lyophilise le produit de collagène isolé.

8. Procédé selon l'une quelconque des revendications 1 à 7, **caractérisé en ce que** l'on dépose la matière d'éponge de départ dans de l'alcool, on lave ensuite avec de l'eau, on ajoute un agent d'extraction à une valeur de pH de 7-12 et on traite l'extrait de collagène résultant.

9. Collagène d'éponges natif présent à un pH de 8-11 dans la couche surnageante d'une centrifugation obtenu selon le procédé selon la revendication 8.

10. Collagène selon la revendication 9 pour une application dans la pharmacie, la médecine, la cosmétique, la chimie agro-alimentaire.

11. Utilisation d'un produit de collagène préparé selon le procédé selon l'une quelconque des revendications 1 à 8 pour la préparation de crèmes, de baumes, de suspensions, de comprimés, de capsules, d'implants, d'emplâtres, de mousses, de supports de matières actives en Parenteralia et Enteralia, de gouttes oculaires, de nanocapsules pour des supports de matières actives, de véhicules pour des matières actives à travers la peau, les muqueuses, les vaisseaux et les membranes d'organes.

12. Procédé pour la préparation de collagène nanoparticulaire à partir d'éponges marines, **caractérisé en ce que** l'on disperse et on homogénéise comme matériau de collagène de départ un produit de collagène préparé selon le procédé selon l'une quelconque des revendications 1 à 8, on émulsionne ensuite et on réticule avec un excès d'agent de réticulation et on traite ensuite.

13. Procédé selon la revendication 12, **caractérisé en ce que** l'on utilise du collagène obtenu à partir des éponges de la classe Chondrosiidae.

14. Procédé selon l'une quelconque des revendications 12 à 13, **caractérisé en ce que** l'on utilise comme agent de réticulation du glutardialdéhyde.

15. Collagène nanoparticulaire à partir d'éponges obtenu selon le procédé selon l'une quelconque des revendications 12 à 14.

16. Collagène selon la revendication 15 pour une application dans la pharmacie, la médecine, les cosmétiques, la chimie agro-alimentaire.

17. Utilisation d'un produit de collagène selon la revendication 15 ou préparé selon les revendications 12-14 pour la préparation de crèmes, de baumes, pour une application sur la peau et les muqueuses, de suspensions, de comprimés, de capsules, d'implants, d'emplâtres, de mousses, d'éponges, de non-tissés, pour recouvrir des plaies, de nanocapsules comme supports de matières actives, de supports de matières actives en Parenteralia et Enteralia, de gouttes oculaires, de véhicules pour des matières actives à travers la peau, les muqueuses, les vaisseaux et les membranes d'organes.

18. Utilisation selon la revendication 17 pour la préparation d'un agent pouvant être administré oralement pour le traitement de maladies de l'appareil circulatoire.

19. Utilisation selon la revendication 17 pour la préparation d'un agent pouvant être administré topiquement dans la forme d'un oléo- ou d'un hydrogel.

20. Utilisation d'un produit de collagène préparé selon les revendications 1-8 ou 12-14 ou du produit de collagène selon la revendication 9 ou 15 comme agent biochimique destiné à l'application de promotion de croissance in vitro pour des cellules permanentes ou neuronales.

21. Gel dans la forme d'un oléogel ou d'un hydrogel contenant de l'eau, une base de gel ainsi que du collagène natif provenant d'éponges selon la revendication 9 ou préparé selon le procédé selon l'une quelconque des revendications 1 à 8 ou du nanocollagène obtenu à partir de celui-ci selon la revendication 15 ou préparé selon les revendications 12 à 14.

22. Suspension de collagène contenant du collagène natif selon la revendication 9 ou préparée selon le procédé selon l'une quelconque des revendications 1 à 8 ou du nanocollagène obtenu à partir de celui-ci selon la revendication 15 ou préparé selon les revendications 12 à 14 dans de l'eau dans une quantité de 1-10 g de collagène/250 ml.
